Europäisches Patentamt

European Patent Office

Office européen des brevets

(11) Veröffentlichungsnummer: **0 071 170**
**A2**

## (12) EUROPÄISCHE PATENTANMELDUNG

(21) Anmeldenummer: **82106568.7**

(22) Anmeldetag: **21.07.82**

(51) Int. Cl.$^3$: **D 01 D 5/24**
C 08 J 9/28, B 01 D 13/04
B 01 D 46/00, G 03 C 1/78
A 61 L 15/03, G 01 N 33/52

(30) Priorität: **28.07.81 DE 3129744**

(43) Veröffentlichungstag der Anmeldung:
**09.02.83 Patentblatt 83/6**

(84) Benannte Vertragsstaaten:
**BE CH DE FR GB IT LI NL SE**

(71) Anmelder: **HOECHST AKTIENGESELLSCHAFT**
**Postfach 80 03 20**
**D-6230 Frankfurt/Main 80(DE)**

(72) Erfinder: **Walch, Axel, Dr.**
**Hans-Sachs-Strasse 5**
**D-6000 Frankfurt am Main(DE)**

(72) Erfinder: **Seifried, Walter, Dr.**
**Am Güldenplan 9**
**D-6200 Wiesbaden 1(DE)**

(72) Erfinder: **Michel, Wolfgang**
**Am Hohen Stein 24**
**D-6200 Wiesbaden 1(DE)**

(72) Erfinder: **Kuhls, Jürgen, Dr.**
**Unghausen 16a**
**D-8263 Burghausen(DE)**

(72) Erfinder: **Wildhardt, Jürgen**
**Am Birnbusch 14**
**D-6274 Hünstetten(DE)**

(54) **Für Flüssigkeiten sowie Gase selektiv-durchlässige Formkörper aus Fluorgruppen enthaltendem Copolymerisat, die zugleich oleophob und oleophil sind.**

(57) Die Erfindung betrifft für Flüssigkeiten und Gase selektiv-durchlässige Folien sowie rohrförmige Gebilde auf Basis eines Copolymerisates, das aus copolymerisiertem fluoriertem Olefin, copolymerisiertem Vinylacetat und gegebenenfalls copolymerisiertem Olefin aufgebaut ist, wobei die Acetatgruppen des Copolymerisates gegebenenfalls zu OH-Gruppen verseift sein können, wobei die Folien bzw. rohrförmigen Gebilde jeweils inhärente latente Strukturumwandelbarkeit besitzen.

Die Erfindung umfaßt ferner Verfahren zur Herstellung der bezeichneten Formkörper sowie Verfahren zu deren Strukturumwandlung.

Die Erfindung betrifft ferner die Verwendung erfindungsgemäßer Formkörper.

BILD I

HOECHST AKTIENGESELLSCHAFT
KALLE Niederlassung der Hoechst AG

Hoe 81/K 042                              19. Juli 1982
                                         WLJ-Dr.Wa-gv

FÜR FLÜSSIGKEITEN SOWIE GASE SELEKTIV DURCHLÄSSIGE FORM-
KÖRPER AUS FLUORGRUPPEN ENTHALTENDEM COPOLYMERISAT, DIE
ZUGLEICH OLEOPHOB UND OLEOPHIL SIND

Die Erfindung betrifft für Flüssigkeiten sowie Gase in originärem Zustand durchlässige Formkörper aus Fluorgruppen enthaltendem synthetischem Copolymerisat, die zugleich oleophob und oleophil sind sowie die Verwendung derartiger Formkörper. Die Erfindung betrifft ferner Verfahren zur Herstellung der angegebenen Formkörper sowie Verfahren zur Umwandlung ihrer Struktur.
Die Erfindung betrifft auch die Verwendung der Formkörper.

Die Bezeichnung "Formkörper" soll im Rahmen der vorliegenden Erfindungsbeschreibung und der Ansprüche Folien sowie rohrförmige Gebilde, wie Schläuche sowie Hohlfäden (Kapillaren), umfassen. Definitionsgemäß umfaßt die Bezeichnung "Formkörper" auch Beschichtungen.

Es sind offenporig-mikroporöse Kunststoffolien bekannt, beispielsweise solche aus Polyamid, Polysulfon oder Polyvinylidenfluorid (US-PS 3,615,024), die nach dem sogenannten Phaseninversionsverfahren durch Gießen einer Kunststofflösung zu einem flüssigen Film und Koagulieren des in diesem gelösten Kunststoffs unter Ausbildung eines formbeständigen mikroporösen Films aus diesem hergestellt sind.
Auch werden in der Europ.Patentanmeldung 0 012 557 offenporige Folien vorgeschlagen, die durch Mischen von Polyvinylidenfluorid mit Polyvinylacetat hergestellt werden.
Erst die fertige Folie wird anschließend durch Verseifen in eine Mischung mit Polyvinylalkohol umgewandelt.
Direkt hydrophil/hydrophobe Copolymerisate des Tetrafluorethylen mit Vinylacetat bzw. Vinylalkohol als selektiv-durchlässige Folien wurden bisher noch nicht eingesetzt.

Der Erfindung liegt die Aufgabe zugrunde, Formkörper vorzuschlagen, die infolge des qualitativen und quantitativen chemischen Aufbaues des sie bildenden Copolymerisates die Eigenschaft besitzen, zugleich oleophob und oleophil zu sein, die dadurch verträglich mit unterschiedlichsten, sie kontaktierenden Bestandteilen sind und deren Durchlässigkeit für Flüssigkeiten und Gase in einem breiten Bereich variabel eingestellt werden kann. Die Formkörper sind auch gegen aggressive chemische Mittel beständig und können auch ohne Änderung ihres Eigenschaftsprofils mit oder ohne Weichmacher getrocknet und äußerst rasch mit unterschiedlichen Medien rückbefeuchtet werden.

Die  für Flüssigkeiten und Gase selektiv-durchlässigen Formkörper sind somit nicht nur verträglich sowohl mit oleophilen als auch oleophoben Fluiden, sondern können außerdem durch einfache Varianten bei ihrer Herstellung entweder mit einer sehr offenporigen oder einer engporigen Struktur versehen werden.

Die der Erfindung zugrundeliegende Aufgabe wird gelöst durch die in Anspruch 1 bis 4 angegebenen Formkörper. Be-

HOECHST AKTIENGESELLSCHAFT
KALLE Niederlassung der Hoechst AG

- 3 -

sondere Varianten der gegenständlichen Erfindung sind in den Ansprüchen 11 bis 13 angegeben. Besonders vorteilhafte Ausgestaltungen der Erfindungen sind jeweils in den Unteransprüchen konkretisiert. Verfahren zur Herstellung der erfindungsgemäßen Formkörper sind in den Ansprüchen 14 bis 17 angegeben. Die Ansprüche 18 und 19 beziehen sich auf Verfahren zur Strukturumwandlung der gegenständlichen Erfindung nach Anspruch 1 bis 10.

Die erfindungsgemäße Folie bzw. die Wandung des erfindungsgemäßen rohrförmigen Gebildes ist originär für Flüssigkeiten sowie Gase, die jeweils das die Folie bzw. das rohrförmige Gebilde aufbauende Copolymerisat nicht anlösen und nicht angreifen, durchlässig; unter der Bezeichnung originäre Folie bzw. originäres rohrförmiges Gebilde wird jeweils eine solche bzw. ein solches verstanden, die bzw. das nach seiner Herstellung keiner Behandlung unterworfen wurde, durch die sie bzw. es für strömende Flüssigkeiten oder Gase durchlässig wird.

Chemisch-stofflich ist der erfindungsgemäße Formkörper dadurch charakterisiert, daß er zu wenigstens 50 Gew.% aus filmbildendem synthetischem Copolymerisat (1) besteht, das zu 20 bis 80 Gew.-%, bezogen auf das Gesamtgewicht desselben, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.-%, bezogen auf das Gesamtgewicht des Copolymerisates, aus

HOECHST AKTIENGESELLSCHAFT
KALLE Niederlassung der Hoechst AG

- 4 -

copolymerisiertem Olefin, bevorzugt copolymerisiertem Ethylen oder copolymerisiertem Propylen und zu 80 bis 20 Gew.-%, bezogen auf das Gesamtgewicht des Copolymerisates, aus copolymerisiertem Vinylacetat aufgebaut ist. Definitionsgemäß werden unter Copolymerisat auch Blockcopolymerisate oder Mischungen derselben verstanden. Der Formkörper kann bis zu 50 Gew.%, bezogen auf sein Gesamtgewicht, Polymerisate enthalten, die sich in ihrem qualitativen chemischen Aufbau von den bezeichneten Copolymerisaten unterscheiden, die zu wenigstens 50 % im Formkörper enthalten sind. Der Formkörper kann beispielsweise wenigstens zu 50 Gew.% aus bezeichnetem Copolymerisat und zu insgesamt bis zu 50 Gew.% aus Polyvinylidenfluorid, polaren Polyolefinen, Silikonen oder Abmischungen dieser Polymeren bestehen.

Der Formkörper kann auch zu wenigstens 50 Gew.-% aus Copolymerisat bestehen, das zu 20 bis 80 Gew.-%, bezogen auf das Gesamtgewicht desselben, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.-%, bezogen auf das Gesamtgewicht des Polymerisates, aus copolymerisiertem Olefin, bevorzugt copolymerisiertem Ethylen oder copolymerisiertem Propylen und zu 80 bis 20 Gew.-%, bezogen auf das Gesamtgewicht des Copolymerisates, aus copolymerisiertem Vinylacetat aufgebaut ist, wobei wenigstens 5 Gew.-%, vorzugsweise mehr als 80 Gew.-%, der Acetatgruppen im angegebenen Copolymerisat, bezogen auf deren Gesamtmenge in diesem, nach erfolgter Copolymerisation der bezeichneten Comonomeren zum angegebenen Copolymerisat durch Verseifung derselben in OH-Gruppen umgewandelt sind.

Es ist auch möglich, das Copolymerisat über seine funktionellen Gruppen mit sich, mit polymeren Beimischungen oder mit multifunktionellen Agentien (z.B. Polyisocyanate) zu vernetzen.

Bevorzugt besteht der Formkörper zu wenigstens 50 Gew.-% aus Copolymerisat (2), das zu 30 bis 70 Gew.-%, bezogen auf sein Gesamtgewicht, aus copolymerisiertem Tetrafluorethylen, zu 0 bis 20 Gew.-%, bezogen auf das Gesamtgewicht des Copolymerisates, aus copolymerisiertem Ethylen und zu 70 bis 30 Gew.-%, bezogen auf das Gesamtgewicht des Copolymerisates aus copolymerisiertem Vinylacetat aufgebaut ist.

Der Formkörper kann auch zu wenigstens 50 Gew.-% aus einem Copolymerisat bestehen, das zu 30 bis 70 Gew-%, bezogen auf sein Gesamtgewicht, aus copolymerisiertem Tetrafluorethylen, zu 0 bis 20 Gew.-%, bezogen auf das Gesamtgewicht des Copolymerisates, aus copolymerisiertem Ethylen und zu 70 bis 30 Gew.-%, bezogen auf das Gesamtgewicht des Copolymerisates, aus copolymerisiertem Vinylacetat aufgebaut ist, wobei mehr als 5 Gew.-%, bevorzugt mehr als 80 Gew.-%, der Gesamtmenge der im bezeichneten Copolymerisat enthaltenen Acetatgruppen, bezogen auf deren Gesamtgewicht, durch Verseifung derselben nach erfolgter Copolymerisation der bezeichneten

HOECHST AKTIENGESELLSCHAFT
KALLE Niederlassung der Hoechst AG

- 6 -

Comonomeren zum angegebenen Copolymerisat, in OH-Gruppen umgewandelt sind.

Vorteilhaft enthält der Formkörper z.B. Copolymerisat (3), das zu 45 Gew.-% aus copolymerisiertem Tetrafluorethylen, zu 7 Gew.-% aus copolymerisiertem Ethylen und zu 48 Gew.-% aus copolymerisiertem Vinylacetat besteht, wobei mehr als 80% der Acetatgruppen des Copolymerisates nach Herstellung desselben zu OH-Gruppen verseift sind.

Ein anderer Formkörper enthält beispielsweise Copolymerisat (4), das zu 58 Gew.-% aus copolymerisiertem Tetrafluorethylen, zu 7 Gew.-% aus copolymerisiertem Ethylen und zu 35 Gew-% aus copolymerisiertem Vinylacetat aufgebaut ist, wobei mehr als 80 % der Acetatgruppen des Copolymerisats nach dessen Herstellung zu OH-Gruppen verseift sind.

Ein besonderer Formkörper enthält beispielsweise ein zweikomponentiges Copolymerisat (5), es ist zu 62 Gew.-%, bezogen auf das Gesamtgewicht des Copolymerisates, aus copolymerisiertem Tetrafluorethylen und zu 38 Gew.-%, aus copolymerisiertem Vinylacetat aufgebaut, wobei mehr als 80% der Acetatgruppen des Copolymerisates nach dessen Herstellung durch Verseifung in OH-Gruppen übergeführt sind.

Die gewichtsprozentualen Angaben zu den in den beispielhaften Copolymerisaten jeweils enthaltenen Mengen bezeichneter copolymerisierter Comonomerer gelten jeweils

HOECHST  AKTIENGESELLSCHAFT
KALLE  Niederlassung der Hoechst AG

- 7 -

mit der Maßgabe, daß deren Gesamtsumme jeweils 100% beträgt.

Copolymerisate des genannten chemischen Aufbaues sind nach Verfahren herstellbar, die dem Fachmann geläufig sind (US-PS 3,445,434 sowie US-PS 2,468,664). Die vorstehend angegebenen Copolymerisate sind per se nicht Gegenstand vorliegender Erfindung.

Infolge des chemischen Aufbaues des den Formkörper bildenden Copolymerisates ist dieser innerhalb seiner ihn bildenden Polymerketten jeweils zugleich oleophob als auch oleophil und auch in aggressiven Medien beständig.

Die erfindungsgemäße Folie bzw. die Wandung des erfindungsgemäßen rohrförmigen Gebildes hat jeweils vorteilhaft eine Dicke im Bereich von 0,5 bis 800 /um. Die Folie kann selbsttragend sein oder sich auf einem Trägerflächengebilde, beispielsweise einer streckorientierten Folie aus Polyester oder einer Folie aus weichmacherfreiem Polyvinylchlorid, befinden.

Die erfindungsgemäße Folie bzw. die Wandung des rohrförmigen Gebildes besitzt in einer ersten Ausführungsform - erste Erfindungsvariante - offenporige Struktur und hat Poren mit effektivem Durchmesser im Bereich von 0,002 bis 10 /um.

In dieser Ausführungsform ist die Folie bzw. die Wandung des rohrförmigen Gebildes von Flüssigkeiten durchström-

HOECHST AKTIENGESELLSCHAFT
KALLE Niederlassung der Hoechst AG

- 8 -

bar, das bedeutet, daß deren Durchtritt durch die Folie
bzw. durch die Wandung des rohrförmigen Gebildes jeweils
auf dem Wege über kommunizierende Poren erfolgt.
Bei Flüssigkeiten werden dabei in diesen gelöste Moleküle
von der Folie bzw. der Wandung des rohrförmigen Gebildes
zurückgehalten, wenn diese größer als die Poren in der
Folie bzw. in der Wandung des rohrförmigen Gebildes sind.

Gemäß der zweiten Ausführungsform des erfindungsgemäßen
Formkörpers (zweite Erfindungsvariante) besitzt dieser
physikalisch praktisch homogene Struktur.

Die Angabe, daß der Formkörper physikalisch praktisch
homogene Struktur besitzt, soll definitionsgemäß bedeuten, daß sein Anteil an freien, lichtbrechenden Poren
vernachlässigbar gering ist.

Bei der zweiten Erfindungsvariante können Flüssigkeiten
sowie Gase nur infolge von Diffusion durch die Folie bzw.
die Wandung des rohrförmigen Gebildes hindurchtreten.
Dabei werden Moleküle, die infolge ihrer Molekülgröße
oder ihrer Löslichkeit nicht zur Diffusion durch die
Folie bzw. die Wandung des rohrförmigen Gebildes befähigt
sind, jeweils von dieser zurückgehalten.

Bei der ersten Erfindungsvariante ist eine Folie bzw. ein
rohrförmiges Gebilde, insbesondere in Form von Hohlfäden
(Kapillaren) jeweils mit Poren von effektivem Durchmesser
im Bereich von 0,05 bis 10 $\mu$m, insbesondere zur Verwendung als Membranfilter zur Trennung grob disperser wäß-

HOECHST AKTIENGESELLSCHAFT
KALLE Niederlassung der Hoechst AG

- 9 -

riger sowie gasförmiger Systeme, beispielsweise zur Entfernung von Blutformkörpern aus Blut (Plasmapherese), zur Trennung von partikulären Verunreinigungen aus Infusionslösungen, zur Trennung von Bakterien oder Viren aus Lösungen oder Gasen (Sterilfilter) oder zur Trennung von Feinstpartikeln bzw. Aerosolen aus Gasen geeignet.

Folien bzw. Hohlfäden der ersten Erfindungsvariante, die Poren mit effektivem Durchmesser im Bereich von 0,002 bis 0,05 ‚um haben, lassen sich vorteilhaft, beispielsweise als Ultrafilter, Hämofilter, zur Trennung oder zur Reinigung von Dispersionen, Emulsionen sowie gelösten Makromolekülen, beispielsweise zur Separation von Milch/Molke oder von Blutbestandteilen (künstliche Nieren), verwenden.
Aufgrund ihrer biologischen Verträglichkeit und ihrer Beständigkeit eignen sich genannte Folien auch als permeable Umhüllungen bzw. Medien zur Aufnahme und nachfolgender Abgabe, beispielsweise von Therapeutika, Katalysatoren oder von sonstigen Wirkstoffen und Substanzen, beispielsweise Freisetzung von Insulin aus membranumhüllten implantierten Zellkulturen.

Formkörper der ersten Erfindungsvariante lassen sich nach Befeuchtung trocknen und danach wiederverwenden; ihr Eigenschaftsprofil wird durch den Trockenvorgang nicht verändert; sie können aber auch nach Einbringung eines Weichmachers (z.B. Glycerin) getrocknet werden.

Formkörper der ersten Erfindungsvariante sind durch in-

HOECHST AKTIENGESELLSCHAFT
KALLE Niederlassung der Hoechst AG

- 10 -

härente, latente Umwandelbarkeit ihrer Struktur charakterisiert. Sie sind als Zwischenprodukte zur Herstellung
physikalisch praktisch homogener Formkörper geeignet, in
die sie durch eine Strukturumwandlungsmaßnahme überführbar sind.

Gebündelt können die angegebenen Hohlfasern als Filterelementeinheit verwendet werden.

Erfindungsgemäße Formkörper sind beispielsweise herstellbar, indem man flüssige Lösung, die 1 bis 50 Gew.%, bevorzugt 5 bis 25 Gew.%, bezogen auf das Gesamtgewicht der
Lösung, Polymeres gelöst enthält, koaguliert, wobei der
gelöste Anteil zu wenigstens 50 Gew.% aus einem der
vorstehend qualitativ und quantitativ chemisch bezeichneten Copolymerisate besteht.
Das Lösungsmittel der Lösung kann beispielsweise Dimethylformamid, N-Methylpyrrolidon, Dimethylsulfoxid, Dimethylacetamid, aliphatischer Alkohol, Aceton oder
Tetrahydrofuran sein.
Die Lösung wird in Form eines flüssigen Filmes aus dem
geraden Schlitzspalt eines Düsenkörpers oder in Form
eines flüssigen Hohlfadens aus dem Ringschlitzspalt eines
Düsenkörpers jeweils aus- und in Fällflüssigkeit einpreßt; vor Eintritt in das Fällbad kann z.B. auch eine
Verweildauer an der Luft vorgeschaltet werden. In der
Fällflüssigkeit ist das in der Lösung gelöste Copolymerisat unlöslich, das Lösungsmittel jedoch löslich.
Als Fällflüssigkeit wird beispielsweise Wasser verwendet.

Die Fällflüssigkeit koaguliert bei ihrer Einwirkung (in

HOECHST AKTIENGESELLSCHAFT
KALLE Niederlassung der Hoechst AG

- 11 -

Abhängigkeit z.B. von der Fällbadtemperatur) auf den flüssigen Film bzw. auf den flüssigen Hohlfaden, das in diesem enthaltene Copolymerisat unter Ausbildung einer formbeständigen Folie bzw. unter Ausbildung eines formbeständigen Hohlfadens aus bezeichnetem Copolymerisat die bzw. der die angegebene selektiv-durchlässige Struktur besitzt.

Der Film bzw. der Hohlfaden wird dann durch Trocknung von überschüssiger Flüssigkeit befreit oder die Fällflüssigkeit durch eine andere Flüssigkeit (z.B. Glycerin) ersetzt.

Eine Folie gemäß der Erfindung kann auch in der Weise hergestellt werden, daß man eine flüssige Schicht aus vorstehend angegebener Copolymerisatlösung auf die Oberfläche einer formstabilen Trägerfolie, beispielsweise in Gestalt eines Metallbandes, aufbringt und dann Fällflüssigkeit auf die auf der Trägerfolie befindliche flüssige Schicht zur Einwirkung bringt und den dabei gebildeten selektiv-durchlässigen Film aus angegebenem Copolymerisat von der Trägerfolie abzieht.

Wenn als Trägerfolie beispielsweise eine streckorientierte Polyesterfolie oder eine Folie aus Hart-PVC verwendet wird, wobei die Trägerfolie beispielsweise eine Dicke im Bereich von 50 bis 100 um besitzt und im übrigen wie vorstehend angegeben verfährt, kann man nach Ausbildung des Copolymerisatfilmes auf der Trägerfolienoberfläche nach Trocknung des Filmes das zweilagige Laminat bestimmungsgemäß verwenden.

Die Herstellung von Folien mit Poren effektiver Größe im Bereich von 0,05 bis 10 $\mu$m ist beispielsweise durchführbar, indem man eine 13 gewichtsprozentige Lösung des Copolymerisates (nach 5) in N-Methylpyrrolidon herstellt, die Lösung auf einer Glasplatte mit einer 100 $\mu$m Rakel ausstreicht und die Polymerschicht nach einer Verweildauer von 4 s an der Luft (bei 21°C) für 20 min in Wasser (von 16°C) koaguliert.

Folien mit Poren effektiver Größe im Bereich von 0,002 bis 0,05 $\mu$m sind beispielsweise herstellbar, indem man 15 Gew.-% des Copolymeren (nach 3) mit 4 Gew.-% Polyvinylpyrrolidon in N-Methylpyrrolidon/Tetrahydrofuran (5:1) löst, die Lösung auf einer Glasplatte mit einer 100 $\mu$m Rakel ausstreicht und die Polymerschicht nach einer Verweildauer von 20 s an der Luft (bei 21°C) für 30 min in Wasser (von 16°C) koaguliert.

Die Herstellung von Hohlfäden, deren Wandung Poren der vorgenannten Größe besitzen, erfolgt unter den vorstehend angegebenen Bedingungen, jedoch mit der Abwandlung, daß die Polymerenlösung durch den Ringschlitzspalt eines Düsenkörpers in das genannte Fällmittel eingepreßt wird. Formkörper, insbesondere Folien, gemäß der zweiten gegenständlichen Erfindungsvariante sind außerdem auch herstellbar, indem man beispielsweise einen wie angegeben hergestellten Formkörper, beispielsweise eine Folie, in einem weiteren Verfahrensschritt einer der nachfolgend genannten physikalischen oder chemischen Maßnahmen oder einer Kombination beider unterwirft, z.B.

HOECHST AKTIENGESELLSCHAFT
KALLE Niederlassung der Hoechst AG

- 13 -

1. Einwirkung von Wärme auf die Folie. Je nach chemischem Aufbau der Folie bzw. des sie bildenden Copolymerisates, wird sie durch Wärmeeinwirkung auf eine Temperatur im Bereich zwischen 50 und 220 $^o$C erhitzt. Dabei erfolgt die Strukturumwandlung der Folie im angegebenen Temperaturbereich jeweils innerhalb einer relativ engen Temperaturzone von weniger als 10 $^o$C und läuft spontan ab.

2. Beaufschlagung der Folie mit gasförmigem oder flüssigem Medium, das befähigt ist, den die Folie bildenden Kunststoff anzulösen, beispielsweise Aceton in flüssiger oder Dampfform. Die Dauer der Strukturumwandlungsmaßnahme hängt von der Konzentration des flüssigen Mediums und von seiner Temperatur ab; die Strukturumwandlung erfolgt praktisch spontan.

Definitionsgemäß soll unter praktisch spontanem Ablauf der Strukturumwandlung verstanden werden, daß diese sich innerhalb eines Zeitraumes von Sekunden vollzieht.

Die Strukturumwandlung der Folie kann auch dadurch erfolgen, daß man Preßkraft auf diese zur Einwirkung bringt.

Die Strukturumwandlung durch Einwirkung von Wärme oder chemischem Medium auf die Folie, wie vorstehend angegeben, kann auch auch dadurch unterstützt werden, daß man diese Einwirkungsmaßnahmen jeweils mit Preßkrafteinwirkung auf die Folie kombiniert.

Bei der Strukturumwandlung des Formkörpers durch Beaufschlagung desselben mit gasförmigen oder flüssigen Medien, welche den diesen bildenden Kunststoff anzulösen vermögen, sind als solche beispielsweise Tetrahydrofuran, niedermolekulare aliphatische Alkohole, wie beispielsweise Äthanol, inbesondere auch Aceton, geeignet. Die bezeichneten chemischen Medien werden bevorzugt in Dampfform auf den Formkörper zur Einwirkung gebracht.

Je nach Intensität und/oder Einwirkungsdauer der Strukturumwandlungsmaßnahmen auf den Formkörper kann dessen Struktur je nach Bedarf mehr oder minder weitgehend im Grenzfall bis zur physikalisch praktisch homogenen Stufe umgewandelt werden. Es kann somit auch eine offenporige Form des Formkörpers gemäß der ersten Variante nur soweit durch die angeführten Maßnahmen in seiner Durchlässigkeit verändert werden, daß dabei ein solcher mit Poren geringeren Durchmessers und damit höherer Selektivität entsteht.

Bei Anwendung von Temperatur als Umwandlungsmaßnahme kann je nach deren Höhe und Dauer ihrer Einwirkung das Ausmaß der Strukturumwandlung gezielt eingestellt werden. Bei ausreichender Temperatureinwirkung erfolgt die Strukturumwandlung praktisch vollständig, bei kurzer nur teilweise.

Bei Verwendung gasförmiger oder flüssiger Medien zur Strukturumwandlung ist es möglich, durch deren Konzentration und die Einwirkungsdauer die Strukturumwandlung zu steuern.

HOECHST AKTIENGESELLSCHAFT
KALLE Niederlassung der Hoechst AG

- 15 -

Die Strukturumwandlung erfindungsgemäßer Formkörper durch Einwirkung von Wärme auf diese, kann beispielsweise derart erfolgen, daß man sie mit heißer Luft hinreichender Temperatur beaufschlagt oder sie der Einwirkung von Ultrarotstrahlung unterwirft. Beispielsweise wird eine Folie, die aus einem angegebenen bevorzugten Copolymerisat besteht, bei dem eine copolymerisierte Komponente copolymerisiertes Ethylen ist, die eine Dicke von beispielsweise 30 $\mu$m hat, einer Wärmeeinwirkung unterworfen, durch die sie auf eine Temperatur von ca. 90$^{\circ}$C erhitzt wird. Die Strukturumwandlung erfolgt dabei innerhalb eines Zeitraumes von ca. 5 s.

Es ist auch möglich, Folien herzustellen, die in einem oder in mehreren segmentären Bereichen dem Aufbau der ersten Erfindungsvariante und in einem oder in mehreren segmentären Bereichen dem Aufbau der zweiten Erfindungsvariante entsprechen.
Zu diesem Zwecke unterwirft man eine Folie gemäß der ersten Erfindungsvariante jeweils nur bereichsweise einer der angegebenen Strukturumwandlungsmaßnahmen.

Es ist auch möglich, die Struktur von Hohlfäden einer Ausbildung gemäß der ersten gegenständlichen Erfindungsvariante, wie vorstehend angegeben, umzuwandeln, indem man sie, beispielsweise mit Wärme oder mit einem der bezeichneten chemischen Medien beaufschlagt.

Der Formkörper gemäß der ersten gegenständlichen Erfindungsvariante erscheint im Grenzfall vollständiger Umwandlung seiner Struktur physikalisch praktisch homogen
und dadurch transparent; die Strukturumwandlung kann
dabei über Zwischenstufen unterschiedlicher Porosität und
damit unterschiedlicher Lichtdurchlässigkeit bis zur
Transparenz des Formkörpers bis zum Grenzfall seiner
praktisch vollständigen Strukturumwandlung durch Wahl der
Bedingungen unter denen man diese vornimmt, durchgeführt
werden.

Mit zunehmender Lichtdurchlässigkeit des Formkörpers
nimmt dessen freie, lichtbrechende Porosität ab.

Der Formkörper gemäß der ersten Erfindungsvariante wird
wegen seiner Umwandelbarkeit in transparenten Zustand als
"latent transparent" bezeichnet.

Bei dem Formkörper gemäß der ersten gegenständlichen Erfindungsvariante sind dessen Poren mit Fluid beladbar,
d.h. mit diesem ausfüllbar.
Die Struktur des Formkörpers bleibt aufgrund seines Aufbaues stabil, wenn seine Poren mit Flüssigkeit ausgefüllt
werden und man diese zu einem späteren Zeitpunkt wieder
aus den Poren vertreibt oder aus diesen verdunsten läßt.

Unter Fluiden sind definitionsgemäß Flüssigkeiten zu verstehen, gegen die das Copolymerisat, das den erfindungsgemäßen Formkörper bildet, beständig ist, so daß er von
diesen praktisch nicht angelöst wird.
Fluide können chemisch einheitliche Flüssigkeiten oder
Lösungen bzw. Gemische aus solchen sein.

Fluide können auch aus Flüssigkeiten bestehen, die chemische Stoffe gelöst enthalten. Das Lösungsmittel ist dabei der Träger für den in ihm gelösten chemischen Wirkstoff, der mit diesem und durch dieses in die Poren des Formkörpers gelangt, wenn man diesen mit dem Fluid beaufschlagt und dadurch seine Poren mit diesem ausfüllt.

Fluide können auch aus Flüssigkeiten bestehen, die chemische Wirkstoffe in dispergierter oder emulgierter Form enthalten; auch in diesem Fall ist die kontinuierliche Phase des Fluids der Träger für das in ihr Emulgierte oder Dispergierte. Der flüssige Träger bewirkt, daß die in ihm enthaltenen Wirkstoffe in die Poren des erfindungsgemäßen Formkörpers gelangen, wenn man den Formkörper gemäß der ersten Erfindungsvariante mit angegebenen Fluiden beaufschlagt.

Ein Beispiel für ein Fluid der erstgenannten Art ist eine organische flüssig-kristalline Phase von 4-Methoxybenzyliden-4'-n-butylanilin (Smp. 21$^\circ$C).

Ein Beispiel für Fluide der zweiten Gattung ist ein in organischer Phase (z.B. Dodecan) gelöstes photosensitives Sulfonamid des o-Naphtochinondiazides.

Ein Beispiel für ein Fluid mit dispergiertem bzw. emulgiertem Wirkstoffanteil ist eine wäßrige Suspension des Schilddrüsenhormones L-Thyroxin.

Fluide können auch zwei oder mehrere chemisch unterschiedliche Stoffe als Wirkstoff gelöst, dispergiert oder emulgiert enthalten.

Definitionsgemäß soll die Bezeichnung "Fluide" im weiteren Sinne auch Pasten sowie Gele umfassen, wobei deren flüssiger Anteil das Fluid im engeren Sinne ist, da nur dieser in die Poren der erfindungsgemäßen Folie eindringt, wenn man diese mit der Paste oder dem Gel beaufschlagt oder in Kontakt bringt.

Geeignete Gele sind beispielsweise solche auf Basis von Agarose; sie können einen Wasseranteil von beispielsweise ca. 300%, bezogen auf das Gesamtgewicht des Gels, enthalten; der wäßrige Anteil des Gels enthält chemischen Wirkstoff, beispielsweise Scopolamin oder Nitroglycerinderivate gelöst.

Zur Beladung der Poren des Formkörpers mit Fluid kann dieser beispielsweise in eine mit Fluid gefüllte Wanne eingetaucht werden; nach Entnahme aus der Wanne kann er von auf seiner Oberfläche befindlichem überschüssigem Fluid befreit werden, beispielsweise durch Abstreifen, Abpressen oder Abwischen desselben.

Man kann die Poren des Formkörpers auch nur innerhalb segmentärer Bereiche desselben mit Fluid beladen.

Dies soll am Beispiel einer Folie gemäß der ersten Erfindungsvariante erläutert werden.
Man geht dazu in folgender Weise vor:

HOECHST AKTIENGESELLSCHAFT
KALLE Niederlassung der Hoechst AG

- 19 -

Man füllt zunächst sämtliche Poren der Folie in vorstehend angegebener Weise mit Fluid. Danach wandelt man die Struktur der Folie innerhalb eines segmentären Bereiches derselben um, indem man beispielsweise Acetondampf auf diese einwirken läßt. Innerhalb des strukturumgewandelten segmentären Folienbereiches ist Fluid in der Folie immobilisiert enthalten.

Zweckmäßig geht man dabei derart vor, daß man die insgesamt mit Fluid beladene Folie vor der Strukturumwandlungsmaßnahme mit einer Maske abdeckt, die in Form und Abmessung der Folie entspricht. Die Maske hat verschieden gestaltete Ausnehmungen, beispielsweise ein Raster aus rechteckigen Öffnungen. Man läßt dann Acetondampf auf die Maske in der Weise einwirken, daß der von ihr nicht abgedeckte Folienbereich mit diesem beaufschlagt wird.

Die Strukturumwandlung des beaufschlagten segmentären Folienbereiches erfolgt bei Verwendung von gesättigtem Acetondampf innerhalb von 30 bis 60 s.

Man entfernt dann die Maske von der Folie und eluiert dann mit einem geeigneten flüssigen Elutionsmittel das Fluid aus den offenen Poren der Folie. Das in den segmentären strukturumgewandelten Bereichen der Folie enthaltende Fluid wird dabei aus diesem nicht eluiert.

Die Folie wird sodann zur Befüllung ihrer Poren mit Fluid in angegebener Weise behandelt. Das im zweiten Verfahrensschritt verwendete Fluid unterscheidet sich chemisch von dem im ersten Verfahrensschritt verwendeten. Danach

HOECHST AKTIENGESELLSCHAFT
KALLE  Niederlassung der Hoechst AG

- 20 -

wandelt man die Struktur der Folie in vorstehend angebener Weise um.
Das Verfahrensprodukt enthält dann zwei Fluide, die sich
chemisch voneinander unterscheiden, jeweils in definierten Bereichen der Folie, beispielsweise zur Inkorporation
zweier an sich unverträglicher Substanzen in eine Folie.

Man kann das Verfahren auch derart durchführen, daß man
nach der ersten Beladung der Folie mit Fluid innerhalb
segmentärer Bereiche derselben, die Strukturumwandlung
derart führt, daß die gesamte Folie strukturumgewandelt
wird. Das Verfahrenserzeugnis ist dann eine transparente
Folie, die innerhalb eines diskreten Bereiches Fluid
immobilisiert enthält.

Wenn man als Fluid beispielsweise flüssige, flüchtige
Aromastoffe oder Lösungen, die Aromastoffe gelöst enthalten, verwendet oder flüssige, flüchtige Insektizide
oder Lösungen, die diese enthalten, einsetzt, sind diese
nach Strukturumwandlung der Folie in dieser temporär immobilisiert; die bezeichneten chemischen Wirkstoffe treten infolge Diffusion langsam aus der physikalisch
praktisch homogenen Folie bzw. den segmentären Bereichen
derselben aus und entwickeln außerhalb derselben die
erwünschte Wirkung, die entsprechend der Diffusionsgeschwindigkeit der Stoffe langzeitig ist. Sofern man als
Fluid ein solches verwendet, das licht- oder wärmeempfindliche chemische Verbindungen enthält oder reaktive
Verbindungen, die unter Einwirkung eines elektrischen
Potentials ihre Struktur bzw. Farbe verändern, kann eine erfindungsgemäße Folie, die derartige Fluide immobilisiert enthält, auf dem Gebiet der Reproduktionstechnik Anwendung finden oder für phototechnische Verfahren eingesetzt werden.

Die Farbe bzw. die Farbintensität von Folienbereichen, die durch Strukturumwandlung in angegebener Weise physikalisch praktisch homogen sowie transparent erscheinen und gegebenenfalls Fluid immobilisiert enthalten, entspricht der Farbe bzw. der Farbintensität der in ihnen enthaltenen Fluide bzw. den farbgebenden chemischen Stoffen in diesen.

Die selektiv durchlässige Folie gemäß der Erfindung zeichnet sich somit nicht nur durch eine molekular-verteilte oleophil/oleophobe Polymerstruktur aus, was ihren Einsatz in unterschiedlichsten - auch aggressiven - Medien erlaubt und beispielsweise unerwünschte Wechselwirkungen (z.B. Belagbildung) minimiert. Die selektivdurchlässige Folie kann aufgrund der ihr zugrundeliegenden Polymerstruktur außerdem in unterschiedlichsten Durchlässigkieten über einen extrem weiten Porenbereich von praktisch homogen bis zu effektiven Porendurchmessern von 10 /um sowohl durch den Gießprozeß als auch durch nachgeschaltete Strukturumwandlung gezielt eingestellt werden.

Nachfolgend werden beispielhaft weitere Verwendungen erfindungsgemäßer Formkörper angegeben, die jeweils am Beispiel der Verwendung einer Folie gemäß der ersten Erfindungsvariante erläutert werden.

HOECHST AKTIENGESELLSCHAFT
KALLE Niederlassung der Hoechst AG

- 22 -

1. Verwendung einer Folie als temporäres Depot und Umhüllung für Drogen, Katalysatoren, Enzyme, Insektizide, Farbstoffe, Flüssigkristalle, Korrosionsinhibitoren oder zur Abdeckung sterilisierter Güter.

Beispiel:
Eine wäßrige gepufferte Suspension mit 50 mg Pilocarpin wird in einen Beutel aus 5 cm$^2$ erfindungsgemäßer Folie, die aus Copolymerisat (3) besteht, eingefüllt und dicht verschlossen. Der Beutel wird dann 30 s einer gesättigten Atmosphäre aus Acetondampf ausgesetzt. Die ursprünglich weiße Folie wird dabei opak-durchscheinend. Dieser Vorgang geht mit einer definierten Verengung der Poren und damit gewünschten Einstellung ihrer Permeabilität einher. Der Beutel wird sodann in 100 ml gerührte Pufferlösung eingebracht und in dieser aufgehängt. Die Kinetik der Drogenabgabe aus dem Beutel in die ihn umgebende Flüssigkeit wird gemessen. Nach einer Anlaufphase stellt sich bei einer Abgabegeschwindigkeit von 16 mg Wirkstoff pro Woche die erforderliche konstante Kinetik 0. Ordnung ein.

2. Verwendung der Folie für analytische und diagnostische Verfahren, wie Immundiffusion, Immunoelektrophorese, Radioimmunoassay, Agglutinationstests sowie Diagnosesticks.

Beispiel:
5 ml einer Antigenlösung aus wäßrigem gepuffertem Humanalbumin (8 µg) werden in ein Probeapplikationsloch in

HOECHST AKTIENGESELLSCHAFT
KALLE Niederlassung der Hoechst AG

- 23 -

eine Copolymerisatfolie nach (5) eingebracht. Die Folie ist in einer Schichtstärke von 400 um auf einer Polyesterfolie (Hostaphan[R] 100 um) aufgetragen und wurde zuvor mit 14% Antihumanalbuminserum (Kaninchen) getränkt. Nach einer Diffusionszeit von 24 Stunden in einer feuchten Kammer wird die Schicht in physiologischer NaCl-Lösung für 48 Stunden eluiert und direkt anschließend mit einem Proteinfarbstoff (0,1% Coomassieblau) angefärbt und in Acetondampf für 60 s fixiert.

Es liegt eine transparente Folie vor, die eine deutlich ausgeprägte radiale Präzipitatzone aufweist und sich in Transmission quantitativ auswerten läßt.

3. Verwendung als reprographischer und optischer Informationsträger, beispielsweise für Fotokopien mit Naß- und Trockentonern (insbesondere für die Overheadprojektion), als Zeichenfolie, als Substrat für reprographische Folien (insbesondere als Träger photoaktiver Substanzen), als Thermopapier (beispielweise für Thermokopierer), als "Blende" zum Abrufen von Texten und optischer Information, zum zeitlich begrenzten Abschirmen lichtempfindlicher Schichten (beispielsweise von Film und Photomaterial), bzw. umgekehrt zum zeitlich begrenzten Belichten von elektrooptischen oder reaktiven Systemen, zum zeitlich definierten Sichtbarmachen von Informationen, Gegenständen, Räumen oder als Indikator von Dämpfen oder Fluiden sowie spezifischer Temperaturen.

Beispiel a):

Eine weiße, 50 um starke Schicht einer Terpolymerisat-

HOECHST AKTIENGESELLSCHAFT
KALLE Niederlassung der Hoechst AG

- 24 -

folie nach (4 bzw. 3) auf Polyesterfolie (Hostaphan 100 $\mu$m) wird auf einem Overheadprojektor mit einem Filzstift beschriftet, der mit Dodecan getränkt ist. Es entsteht ein transparentes bzw. die Farbe der Folie wiedergebendes, scharf umrissenes Schriftbild auf weißem Untergrund, das nach Übermittlung der gewünschten Information nach wenigen Minuten verschwindet, so daß die Folie erneut einsetzbar ist.

Beispiel b):

Eine weiße, 30 $\mu$m starke Schicht aus Terpolymerisatfolie nach (5) wird auf einer Photokopiermaschine (Infotec 1801) nach einer technischen Testvorlage naß getont und anschließend thermisch bei etwa 90°C fixiert.

Es entsteht eine kratzfeste transparente Folie, die sich durch sehr hohes Auflösungsvermögen der Testlinien auszeichnet und als Vorlage für die Overheadprojektion geeignet ist.

4. Verwendung als Membran- oder Ultrafilter zur Aufarbeitung (Trennung, Konzentrierung, Reinigung) von Aerosolen, Dispersionen, Emulsionen, gelösten oder kolloidalen Makromolekülen in wäßrigen oder gasförmigen Systemen, beispielsweise zur Trennung physiologischer Flüssigkeiten wie Blut (Hämofiltration, Plasmapherese usw.), Milch oder Molke, bzw. Lösungen aus fermentativen Prozessen:

Eine Membran aus Copolymerisat nach (3) wird mit einer Rakel (Spalthöhe 150 $\mu$m) aus einer 20 Gew.-%igen Polymerlösung in Dimethylacetamid auf einer Glasplatte gezo-

gen und in 40 °C warmem Wasser koaguliert. Nach einer Verweildauer von 15 Minuten im Fällbad wird die Membran mit 40 Gew.-%igem Glycerin behandelt und für 10 Minuten bei 100 °C getrocknet. Die 60 um starke Membran wird von der Glasplatte abgelöst und ihre Permeabilität in einer gerührten Testzelle bei 1 bar geprüft: ihr Volumenfluß gegenüber Wasser beträgt 6000 l/m$^2$d, ihr Rückhaltevermögen gegenüber dispersem Polyacrylat-Durchmesser 0,2 um ($^R$Plextol, Röhm & Haas) beträgt 100 %, ihr Rückhaltevermögen gegenüber Polyacrylsäure 60000 Dalton beträgt 40 %.

Da die erfindungsgemäßen Folien bzw. rohrartigen Gebilde schweißbar sind, lassen sich aus ihnen in einfacher Weise Folien- bzw. Schlauchbeutel herstellen; dazu geht man beispielsweise in der Weise vor, daß man zwei Folienstücke jeweils gleicher Form und Abmessung derart übereinanderlegt, daß ihre Ränder fluchten. Die Folienstücke werden dann im Bereich ihrer Ränder miteinander verschweißt. Dabei wird zunächst ein an einer Seite offener Beutel gebildet, dieser wird mit Füllgut befüllt und sodann in angegebener Weise verschlossen.
Bei Verwendung eines Schlauchstückes wird dieses zunächst an seinem einen Ende verschlossen, sein Hohlraum wird dann mit Füllgut befüllt, danach wird das Schlauchstück auch am anderen Ende verschlossen; das Verschließen erfolgt jeweils durch Verschweißung.

Die gegenständliche Erfindung wird durch die Bilder I und II jeweils beispielhaft erläutert.

HOECHST AKTIENGESELLSCHAFT
KALLE Niederlassung der Hoechst AG

- 26 -

Bild I    zeigt eine Photographie einer Folie gemäß der gegenständlichen ersten Erfindungsvariante im Querschnitt in 300-facher Vergrößerung.

Bild II   zeigt eine Photographie einer Folie mit physikalisch praktisch homogener Struktur, die durch Strukturumwandlung einer im Bild I dargestellten Folie hergestellt ist.

In Bild I bedeuten    1 eine Trägerfolie,
                      2 die poröse Folie,
                      3 die freiliegende Oberfläche der Folie 2
                und   4 die Grenzfläche zwischen poröser Folie 2 und Trägerfolie 1.

In Bild II bedeutet   5 eine Folie mit physikalisch praktisch homogener Struktur,
                      6 ist ihre Oberfläche; die Folie 5 ist im von der Kunststoff-Trägerfolie 7 praktisch abgelösten Zustand dargestellt.

Die Bezeichnung - "flüssiger Film" bzw. "flüssiges rohrförmiges Gebilde soll im Rahmen der Erfindungsbeschreibung und den Ansprüchen jeweils die Bedeutung "Film aus Flüssigkeit" bzw. "rohrförmiges Gebilde aus Flüssigkeit" haben.

------

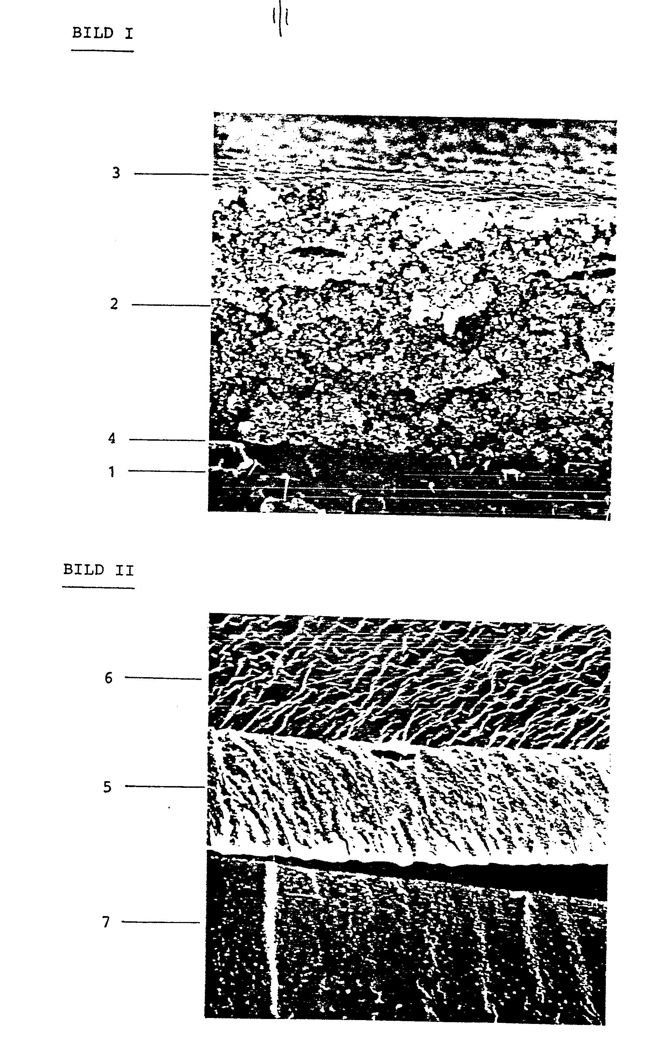

Patentansprüche

1. Für Flüssigkeiten und Gase selektiv durchlässiger Formkörper auf Basis eines Fluorgruppen enthaltenden Copolymerisats, dadurch gekennzeichnet, daß er zu wenigstens 50 Gew.-% aus Copolymerisat besteht, das zu 20 bis 80 Gew.%, bezogen auf das Gesamtgewicht des Copolymerisates, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.%, bezogen auf sein Gesamtgewicht, aus copolymerisiertem Olefin und zu 80 bis 20 Gew.%, bezogen auf das Gesamtgewicht des Copolymerisates, aus copolymerisiertem Vinylacetat aufgebaut ist.

2. Formkörper nach Anspruch 1, dadurch gekennzeichnet, daß das ihn bildende Copolymerisat zu 30 bis 70 Gew.%, bezogen auf dessen Gesamtgewicht aus copolymerisiertem Tetrafluorethylen, zu 0 bis 20 Gew.% aus copolymerisiertem Ethylen und zu 70 bis 30 Gew.%, bezogen auf das Gesamtgewicht des Copolymerisates, aus copolymerisiertem Vinylacetat besteht.

3. Formkörper nach Anspruch 1, dadurch gekennzeichnet, daß das ihn bildende Copolymerisat zu 20 bis 80 Gew.%, bezogen auf das Gesamtgewicht desselben, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.%, bezogen auf das Gesamtgewicht des Copolymerisats, aus copolymerisiertem Olefin, bevorzugt copolymerisiertem Ethylen oder copolymerisiertem Propylen und zu 80 bis 20 Gew.%, bezogen auf das Gesamtgewicht des Copolymerisates, aus copolymerisiertem Vinylacetat aufgebaut ist, wobei wenigstens 5 Gew.%, vorzugsweise mehr als 80 Gew.%, der Acetatgruppen im angegebenen Copolymerisat, bezogen auf deren Gesamtmenge in diesem, nach erfolgter Copolymerisation der bezeichneten Comonomeren zum angegebenen Copolymerisat durch Verseifung derselben in OH-Gruppen umgewandelt sind.

4. Formkörper nach Anspruch 2, dadurch gekennzeichnet, daß das ihn bildende Copolymerisat zu 30 bis 70 Gew.%, bezogen auf sein Gesamtgewicht, aus copolymerisiertem Tetrafluorethylen, zu 0 bis 20 Gew.%, bezogen auf das Gesamtgewicht des Copolymerisats, aus copolymerisiertem Ethylen und zu 70 bis 30 Gew.%, bezogen auf das Gesamtgewicht des Copolymerisates, aus copolymerisiertem Vinylacetat aufgebaut ist, wobei mehr als 5 Gew.%, bevorzugt mehr als 80 Gew.%, der Gesamtmenge der im bezeichneten Copolymerisat enthaltenen Acetatgruppen, bezogen auf deren Gesamtgewicht, durch Verseifung derselben nach erfolgter Copolymerisation der bezeichneten Comonomeren zum angegebenen Copolymerisat, in OH-Gruppen umgewandelt sind.

5. Formkörper nach einem der Ansprüche 1 bis 4, dadurch gekennzeichnet, daß er aus einer Folie besteht, die effektive Poren eines Durchmessers im Bereich von 0,002 bis 10 /um  besitzt und für Flüssigkeiten und Gase durchströmbar ist und inhärente latente Strukturumwandelbarkeit bis zum Grenzfall physikalisch praktischer Homogenität der Folie besitzt.

6. Formkörper nach einem der Ansprüche 1 bis 4, dadurch gekennzeichnet, daß er in Gestalt eines Hohlfadens vorliegt, dessen Wand effektive Poren eines Durchmessers im Bereich von 0,002 bis 10 /um besitzt und die für Flüssigkeiten und Gase durchströmbar ist und inhärente latente Strukturumwandelbarkeit bis zum Grenzfall physikalisch praktischer Homogenität der Wandung besitzt.

7. Formkörper nach einem der Ansprüche 1 bis 4, dadurch gekennzeichnet, daß er aus einer Folie besteht, die effektive Poren eines Durchmessers im Bereich von 0,002 bis 0,05 /um besitzt und für Flüssigkeiten und Gase durchströmbar ist und inhärente latente Strukturumwandelbarkeit bis zum Grenzfall physikalisch praktischer Homogenität besitzt.

8. Formkörper nach einem der Ansprüche 1 bis 4, dadurch gekennzeichnet, daß er aus einem Hohlfaden besteht, dessen Wandung effektive Poren eines Durchmessers im Bereich von 0,002 bis 0,05 /um besitzt und für Flüssigkeiten und Gase durchströmbar ist und inhärente latente Strukturumwandelbarkeit bis zum Grenzfall physikalisch praktischer Homogenität der Wandung besitzt.

HOECHST AKTIENGESELLSCHAFT
KALLE Niederlassung der Hoechst AG

- 30

9. Formkörper nach einem der Ansprüche 1 bis 4, dadurch gekennzeichnet, daß er aus einer Folie besteht, die effektive Poren eines Durchmessers im Bereich von 0,05 bis 10 /um besitzt für Flüssigkeiten sowie Gase durchströmbar ist und inhärente latente Strukturumwandelbarkeit bis zum Grenzfall physikalisch praktischer Homogenität besitzt.

10. Formkörper nach einem der Ansprüche 1 bis 4, dadurch gekennzeichnet, daß er aus einem Hohlfaden besteht, dessen Wandung effektive Poren eines Durchmessers im Bereich von 0,05 bis 10 /um besitzt und für Flüssigkeiten und Gase durchströmbar ist und inhärente latente Strukturumwandelbarkeit bis zum Grenzfall physikalisch praktischer Homogenität der Wandung besitzt.

11. Formkörper nach einem der Ansprüche 1 bis 4, dadurch gekennzeichnet, daß er physikalisch praktisch homogene Struktur besitzt, für Flüssigkeiten sowie Gase infolge von Diffusion durchlässig und transparent ist.

12. Formkörper nach Anspruch 11, dadurch gekennzeichnet, daß er aus einer Folie besteht.

13. Formkörper nach Anspruch 11, dadurch gekennzeichnet, daß er aus einem Hohlfaden besteht.

14. Verfahren zur Herstellung von Formkörpern nach Anspruch 1 bis 13, dadurch gekennzeichnet, daß man aus flüssiger Lösung, die als gelösten Anteil 1 bis 50 Gew.%,

bevorzugt 5 bis 25 Gew.%, jeweils bezogen auf das Gesamtgewicht der Lösung, Polymeres enthält, das zu wenigstens 50 Gew.%, bezogen auf das Gesamtgewicht des gelösten Anteils der Lösung, aus Copolymerisat besteht, das zu 20 bis 80 Gew.%, bezogen auf das Gesamtgewicht des Copolymerisats, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.%, bezogen auf sein Gesamtgewicht, aus copolymerisiertem Olefin und zu 80 bis 20 Gew.%, bezogen auf sein Gesamtgewicht, aus copolymerisiertem Vinylacetat aufgebaut ist, wobei wenigstens 5 Gew.%, vorzugsweise mehr als 80 Gew.%, der Acetatgruppen im angegebenen Copolymerisat, bezogen auf deren Gesamtmenge in diesem, nach erfolgter Copolymerisation der bezeichneten Comonomeren zum angegebenen Copolymerisat durch Verseifung derselben in OH-Gruppen umgewandelt sind, einen flüssigen Film oder einen flüssigen Hohlfaden bildet, auf diesen jeweils Fällflüssigkeit zur Einwirkung bringt, durch die das in ihm gelöste Copolymerisat zu einer porös strukturierten formstabilen Folie bzw. einem porös strukturierten formstabilen Hohlfaden koaguliert wird.

15. Verfahren gemäß Anspruch 14, dadurch gekennzeichnet, daß insgesamt bis zu 50 Gew.% des in der Lösung gelösten Polymeren aus einem oder mehreren miteinander mischbaren Polymeren bestehen, die sich in ihrem qualitativen chemischen Aufbau von dem bezeichneten Copolymerisat unterscheiden, aus dem die Gesamtmenge des in der

HOECHST AKTIENGESELLSCHAFT
KALLE Niederlassung der Hoechst AG

- 32 -

Lösung gelösten zu wenigstens 50 % besteht, wobei die
Polymeren ausgewählt sind aus einer Gruppe umfassend
Polyvinylidenfluorid, polares Polyolefin, Silikone sowie
Abmischungen dieser Polymeren.

16. Verfahren nach Anspruch 14 zur Herstellung von
Folien bzw. Hohlfäden nach einem der Ansprüche 1 bis 10,
dadurch gekennzeichnet, daß man als Polymeren-Lösung eine
solche einsetzt, die als gelösten Anteil Copolymerisat,
das zu 30 bis 70 Gew.%, bezogen auf sein Gesamtgewicht,
aus copolymerisiertem Tetrafluorethylen, zu 0 bis 20
Gew.%, bezogen auf sein Gesamtgewicht, aus copolymerisiertem Ethylen und zu 70 bis 30 Gew.%, bezogen auf sein
Gesamtgewicht, aus copolymerisiertem Vinylacetat, besteht, in einer Menge im Bereich von 1 bis 50 Gew.%,
bezogen auf das Gesamtgewicht der Lösung, enthält, einen
flüssigen Film bzw. einen flüssigen Hohlfaden bildet, auf
diesen jeweils Fällflüssigkeit zur Einwirkung bringt,
durch die das in ihm gelöste Copolymerisat zu einer porös
strukturierten, formstabilen Folie bzw. einem porös
strukturierten formstabilen Hohlfaden koaguliert wird.

17. Verfahren nach Anspruch 14, dadurch gekennzeichnet,
daß die Copolymerisatlösung als gelösten Anteil ein Copolymerisat enthält, das zu 20 bis 80 Gew.-%, bezogen auf
sein Gesamtgewicht, aus copolymerisiertem fluoriertem
Olefin, bevorzugt copolymerisiertem fluoriertem Ethylen
oder copolymerisiertem fluoriertem Propylen, insbesondere
jedoch aus copolymerisiertem perfluoriertem Ethylen, zu 0
bis 40 Gew.%, bezogen auf sein Gesamtgewicht, aus copolymerisiertem Olefin und zu 80 bis 20 Gew.%, bezogen auf sein Gesamtgewicht, aus copolymerisiertem Vinylacetat aufgebaut ist, wobei wenigstens 5 Gew.-%, vorzugsweise mehr als 80 %, der Acetatgruppen im angegebenen Copolymerisat, bezogen auf deren Gesamtmenge in diesem, nach erfolgter Copolymerisation der genannten Comonomeren zum bezeichneten Copolymerisat, durch Verseifung in OH- Gruppen umgewandelt sind.

18. Verfahren gemäß Anspruch 17, dadurch gekennzeichnet, daß das Copolymerisat mit sich selbst, mit polymeren Abmischungen oder multifunktionellen Agentien vernetzt wird.

19. Verfahren nach Anspruch 16, dadurch gekennzeichnet, daß die Copolymerisatlösung als gelösten Anteil Copolymerisat enthält, das zu 30 bis 70 Gew.-%, bezogen auf sein Gesamtgewicht, aus copolymerisiertem Tetrafluorethylen, zu 0 bis 20 Gew.-%, bezogen auf sein Gesamtgewicht, aus copolymerisiertem Ethylen und 30 bis 70 Gew.-%, bezogen auf sein Gesamtgewicht, aus copolymerisiertem Vinylacetat besteht, wobei wenigstens 5 Gew.-%, vorzugsweise mehr als 80 Gew.-%, der Acetatgruppen im angegebenen Copolymerisat, bezogen auf deren Gesamtmenge in diesem, nach erfolgter Copolymerisation der genannten Comonomeren zum bezeichneten Copolymerisat durch Verseifung in OH-Gruppen umgewandelt sind.

20. Verfahren zur Umwandlung der Struktur von Formkörpern entsprechend einem der Ansprüche 1 bis 10 in

HOECHST AKTIENGESELLSCHAFT
KALLE Niederlassung der Hoechst AG

- 34 -

solche einer Ausbildung gemäß einem der Ansprüche 11 bis 13, bei dem man sie der Einwirkung von Wärme unterwirft oder auf sie chemische Medien in flüssiger oder Gasform einwirken läßt, die befähigt sind, die Struktur des sie bildenden Copolymerisates physikalisch bzw. optisch homogen zu machen oder Druck auf sie zur Einwirkung bringt oder sie zugleicher Einwirkung von Wärme und Druck oder zugleicher Einwirkung von bezeichneten chemischen Medien und Druck oder Wärme unterwirft.

21. Verfahren nach Anspruch 19, dadurch gekennzeichnet, daß man den Formkörper mit chemischem Medium in Dampfform beaufschlagt.

22. Verwendung eines Formkörpers nach Anspruch 1 bis 8 als Ultrafilter, Hämofilter, zur Trennung oder zur Reinigung von Dispersionen, Emulsionen sowie gelösten Makromolekülen, beispielsweise zur Separation von Milch/Molke, von Blutbestandteilen (Künstliche Niere), von Nucleinsäuren oder von Enzymen.

23. Verwendung eines Formkörpers nach Anspruch 1 bis 6 sowie 9 und 10 als Membranfilter zur Trennung grob disperser wäßriger sowie gasförmiger Systeme, beispielsweise zur Entfernung von Blutformkörpern aus Blut (Plasmapherese), zur Trennung von partikulären Verunreinigungen aus Infusionslösungen, zur Trennung zellulärer Bestandteile aus Fermentationslösungen, zur Trennung von Bakterien oder Viren aus Lösungen oder Gasen (Sterilfilter) oder zur Trennung von Feinstpartikeln bzw. Aerosolen aus Gasen.

24. Verwendung einer Folie nach einem der Ansprüche 1 bis 10 als Depot und Umhüllung.

25. Verwendung einer Folie nach einem der Ansprüche 1 bis 10 für analytische und diagnostische Verfahren.

26. Verwendung einer Folie nach einem der Ansprüche 1 bis 10 als reprographischer oder optischer Informationsträger bzw. Informationsvermittler.

-----

BILD I

BILD II

Hoe 81/K 042 - HOECHST AKTIENGESELLSCHAFT